# EUROPEAN PATENT APPLICATION

(11) **EP 2 088 430 A1**
(43) Date of publication of application: **12.08.2009**
(21) Application number: 09250211.1
(22) Date of filing: 27.01.2009
(51) Int. Cl.: G01N 33/543, G01N 27/00, B01L 3/00

(54) **Biosensor having 3D metalic nanowire electrodes forming nanochannel, manufacturing method thereof, and bio disk system having same**

(30) Priority: 30.01.2008 KR 20080009649
(71) Applicant: KOREA INSTITUTE OF SCIENCE AND TECHNOLOGY, Seongbuk-gu Seoul (KR)
(72) Inventor: Lee, Byung Chul, Seoul (KR); Moon, Sung Wook, Namyangju-si Gyeonggi-do (KR)
(74) Representative: Powell, Timothy John

(57) **Abstract**

A biosensor includes an upper substrate having a plurality of metallic nanowires on a lower surface thereof and an injection opening, into which a sample containing biomolecules is injected, a lower substrate having a plurality of metallic nanowires on an upper surface thereof; and a support member supporting the upper and the lower substrates, respectively, with a gap to form a nano channel. The metallic nanowires on the upper substrate and the lower substrate form 3D metallic nanowires electrodes.

## Description

### Field of the Invention

The present invention relates to a biosensor having 3D metallic nanowires electrodes forming nano channel, a manufacturing method thereof, and a bio disk system having the biosensor and, more particularly, to a method for manufacturing 3D metallic nanowires electrodes forming nano channel by forming metallic nanowires on an upper substrate and a lower substrate, aligning the substrates by using the nanowires, and bonding the aligned substrates; a biosensor manufactured by the method; and a bio disk system for detecting biomolecules by using the biosensor.

### Background of the Invention

There has been increasing interest over the last few decades in an extension of human life expectancy and an early detection of disease. Nanobiotechnology, one of cutting edge fusion technology, emerged as a means for realizing the extension of human life expectancy and the early detection of disease. A nano biosensor or nano biochip is a core technology of the nanobiotechnology. There have been attempts to increase sensitivity, accuracy, and so forth of the nano biosensor in the developed countries. Therefore, to increase the sensitivity and the accuracy, a channel where biomolecules to be detected are transferred should have nanometer size as well as a sensor to detect the biomolecules. In particular, nanogap or nanowire can be used for more effective detection, thereby enabling to make high sensitivity sensor.

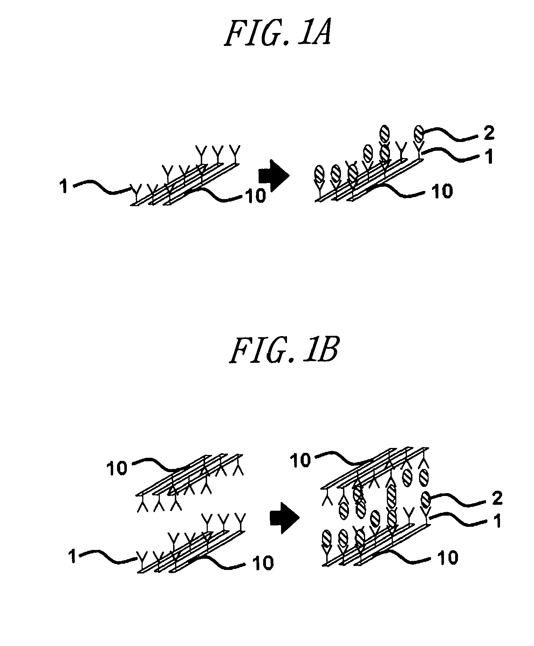

Fig. 1A shows schematic diagram of metallic nanowires electrodes, formed in a planar shape, for use in conventional biosensor. As shown in Fig. 1A, conventional biosensors, in which antibodies 1 are fixed to the nanowires arranged on a substrate in a planar shape, detects the biomolecules by measuring antigen and antibody reaction when a sample containing antigen 2 is inserted thereinto. However, in this case, the probability that the antigen is fixed to the antibody is low. Further, when the concentration of the antigen in the sample is low, the detection range is narrowed, resulting in low sensitivity. That is, conventional nano biosensors are not suitable for high sensitive and efficient detection. Since the contact probability of the biomolecules with a sensing part of the conventional sensors for detecting the biomolecules is very low, large quantity of the sample containing the biomolecules is required when the conventional sensors are used.

### Disclosure of the Invention

In view of the above-noted problems, it is an object of the present invention to provide a method for manufacturing 3D metallic nanowires electrodes forming nano channel by forming metallic nanowires on an upper substrate and a lower substrate, aligning the substrates by using the wires, and bonding the aligned substrates; a biosensor manufactured by the method; and a bio disk system for detecting biomolecules by using the biosensor.

In accordance with one aspect of the present invention, there is provided a biosensor including: an upper substrate having a plurality of metallic nanowires on a lower surface thereof and an injection opening, into which a sample containing biomolecules is injected; a lower substrate having a plurality of metallic nanowires on an upper surface thereof; and a support member supporting the upper and the lower substrates, respectively, with a gap to form a nano channel, wherein the metallic nanowires on the upper substrate and the lower substrate form 3D metallic nanowires electrodes.

In accordance with another aspect of the present invention, there is provided a biosensor including: an upper substrate having a metallic electrode on a lower surface thereof and an injection opening, into which a sample containing biomolecules is injected; a lower substrate having a metallic electrode on an upper surface thereof; and a support member supporting the upper and the lower substrates, respectively, with a gap to form a nano channel, wherein one of electrodes on the upper substrate and the lower substrate is formed with metallic nanowires, and wherein the metallic nanowires on the upper substrate and the lower substrate form 3D metallic nanowires electrodes. In accordance with still another aspect of the present invention, there is provided a method for manufacturing a biosensor including: forming a metallic electrode on an upper surface of a lower substrate; forming a nano channel pattern in a resist coated onto a lower surface of an upper substrate, determining a width and a length of the nano channel; etching a nano channel through the nano channel pattern as a mask; forming a metallic electrode on the etched nano channel; aligning the upper substrate with the lower substrate by using the metallic electrodes on the upper substrate and the lower substrate; and bonding the aligned upper substrate and lower substrate.

In accordance with still another aspect of the present invention, there is provided a method for manufacturing a biosensor including: forming metallic nanowires on an upper surface of a lower substrate; forming metallic nanowires on a lower surface of an upper substrate; spin-coating a polymer on the upper surface of the lower substrate to form a nano channel; etching the polymer to form a nano channel through a mask after determining a width and a length of the nano channel; aligning the upper substrate with the lower substrate by using the metallic nanowires on the upper substrate and the lower substrate; and bonding the aligned upper substrate and lower substrate.

In accordance with still another aspect of the present invention, there is provided a method for manufacturing a biosensor including: forming metallic nanowires on an upper surface of a lower substrate; forming metallic nanowires on a lower surface of an upper substrate; spin-coating a polymer on the upper surface of the lower substrate to form a nano channel; aligning the upper substrate with the lower substrate by using the metallic nanowires on the upper substrate and the lower substrate; bonding the aligned upper substrate and lower substrate; and etching the polymer to form a nano channel through a mask after determining a width and a length of the nano channel.

In accordance with still another aspect of the present invention, there is provided a bio disk system including the above mentioned biosensor to detect biomolecules from an injected sample.

In accordance with still another aspect of the present invention, there is provided a bio disk system, wherein a biosensor is disposed on a thin disk type body of CD-ROM, bio CD, or bio DVD..

The biosensor having 3D metallic nanowires electrodes manufactured by forming metallic nanowires on the upper and lower substrates, aligning the upper and lower substrates by using the metallic nanowires, and bonding the aligned substrates enables real time electric signal detection, thereby performing high sensitivity and high efficiency detection. The biosensor also enables to increase a probability that the biomolecules contacts with the sensing part for detecting the biomolecules so that biomolecules from a low concentration sample or a small amount of sample can be detected. Further, a bio disk system using the biosensor may be economically manufactured and also may be easily manufactured at home using conventional optical disk, thereby providing self diagnostic method in home use.

### Brief Description of the Drawings

The above and other objects and features of the present invention will become apparent from the following description of embodiments given in conjunction with the accompanying drawings, in which:
Fig. 1A shows schematic diagram of metallic nanowires electrodes, formed in a planar shape, for use in conventional biosensor;
Fig. 1B shows schematic diagram of 3D metallic nanowires electrodes for a nano biosensor in accordance with the present invention;
Figs. 2A and 2B are cross sectional view and plan view showing a biosensor having 3D metallic nanowires electrodes in accordance with first embodiment of the present invention;
Figs. 3A and 3B are cross sectional view and plan view showing a biosensor having 3D metallic nanowires electrodes in accordance with second embodiment of the present invention;
Figs. 4A and 4B are cross sectional view and plan view showing a biosensor having 3D metallic nanowires electrodes in accordance with third, embodiment of the present invention;
Figs. 5A and 5B are cross sectional view and plan view showing a biosensor having 3D metallic nanowires electrodes in accordance with fourth embodiment of the present invention;
Figs. 6A and 6B are cross sectional view and plan view showing a biosensor having 3D metallic nanowires electrodes in accordance with fifth embodiment of the present invention;
Fig. 7 is a flow diagram showing a manufacturing method of a biosensor having 3D metallic nano wires electrodes in accordance with an embodiment of the present invention;
Fig. 8 is a flow diagram showing a manufacturing method of a biosensor having 3D metallic nano wires electrodes in accordance with another embodiment of the present invention;
Fig. 9 is a flow diagram showing a manufacturing method of a biosensor having 3D metallic nano wires electrodes in accordance with still another embodiment of the present invention;
Fig. 10A is a schematic diagram of a bio disk system having the biosensor manufactured in accordance with the present invention;
Fig. 10B is a schematic diagram of a biosensor having the 3D metallic nanowires electrodes manufactured in accordance with the present invention; and
Fig. 10C is a plan view showing the bio disk system shown in Fig. 10A arranged on a disk type body.

### Detailed Description of the Embodiments

Embodiments of the present invention will now be described with reference to accompanying drawings which form a part hereof.

Fig. 1B shows schematic diagram of 3D metallic nanowires electrodes for a nano biosensor in accordance with the present invention.

Referring to Fig. 1B, in the biosensor having 3D metallic nanowires electrodes for a nano biosensor in accordance with the present invention, metallic nanowires 10 are arranged above the other nanowires 10 to form a gap therebetween. Accordingly, the probability that antigens are fixed to the antibodies is increased with the gap between the upper and lower metallic nanowires 10 (hereinafter, referred to 3D metallic nanowires gap) as well as gaps between respective nanowires in planar shape. Further, a number of electrodes for electrical detection are increased, thereby enhancing sensitivity.

In particular, in case of detection using the biosensor having the 3D metallic nanowires electrodes in accordance with the present invention, measuring methods of resistance, capacitance, inductance, or impedance between the upper and lower metallic nanowires 10 can be used as well as between the respective nanowires in planar shape, thereby enhancing sensitivity.

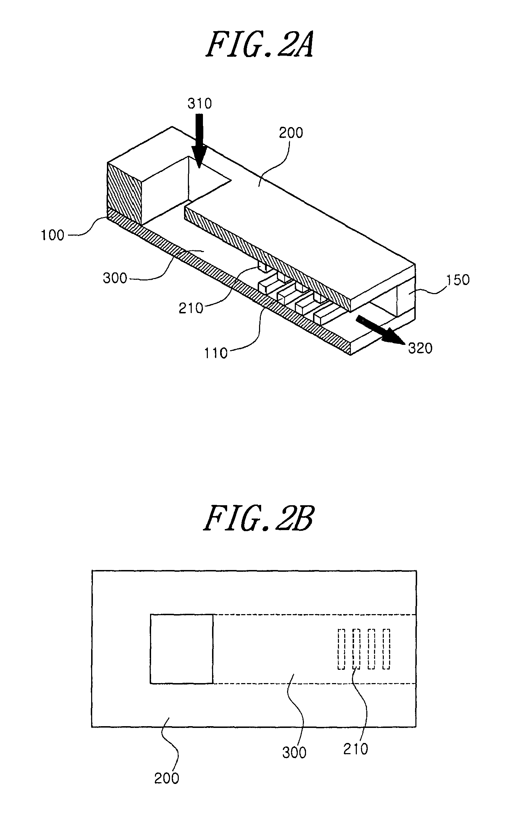

Figs. 2A and 2B are cross sectional view and plan view showing a biosensor having 3D metallic nanowires electrodes in accordance with first embodiment of the present invention.

Referring to Fig. 2A, the biosensor having 3D metallic nanowires electrodes in accordance with the first embodiment of the present invention includes an upper substrate 200 having a plurality of metallic nanowires 210 on a lower surface thereof and an injection opening 310, in which a sample containing biomolecules is injected; a lower substrate 100 having a plurality of metallic nanowires 110 on an upper surface thereof; and a support member 150 supporting the upper and the lower substrates 200 and 100, respectively, with a gap to form a nano channel 300.

The metallic nanowires 210 and 110 are made of low resistance metal such as Ag, Cu, Au, Al, and Pt or an alloy containing one of those metals. Further, between the planar upper and lower substrates 200 and 100, the support member 150 may be made of material different from that of the upper and lower substrates 200 and 100, e.g., a polymer or may be integrally formed with the upper and lower substrates 200 and 100.

As shown in Fig. 2B, the metallic nanowires 210 and 110 formed onto the upper and lower substrates 200 and 100 are vertically disposed to the nano channel 300 and they are aligned with each other. That is, the metallic nanowires 110 of the lower substrate 100 are aligned with the metallic nanowires 210 of the upper substrate 200 to be covered by the metallic nanowires 210 when seen from the top.

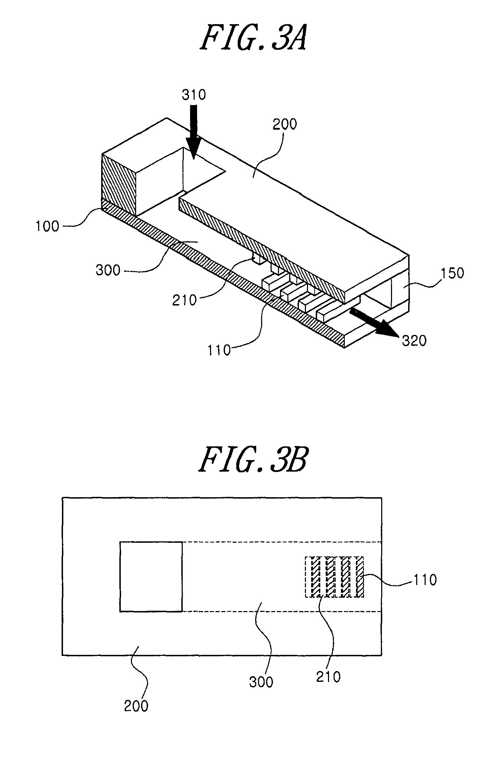

Figs. 3A and 3B are cross sectional view and plan view showing a biosensor having 3D metallic nanowires electrodes in accordance with second embodiment of the present invention.

Referring to Fig. 3A, the biosensor having 3D metallic nanowires electrodes in accordance with the second embodiment of the present invention includes, as like the first embodiment of the present invention, an upper substrate 200 having a plurality of metallic nanowires 210 on a lower surface thereof and an injection opening 310, into which a sample containing biomolecules is injected; a lower substrate 100 having a plurality of metallic nanowires 110 on an upper surface thereof; and a support member 150 supporting the upper and the lower substrates 200 and 100, respectively, with a gap to form a nano channel 300.

As shown in Fig. 3B, the metallic nanowires 210 and 110 formed onto the upper and lower substrates 200 and 100 are vertically disposed to the nano channel 300 and they are alternately aligned with each other. That is, the metallic nanowires 110 of the lower substrate 100 are aligned with the metallic nanowires 210 of the upper substrate 200 to be exposed through gaps between the metallic nanowires 210 when seen from the top.

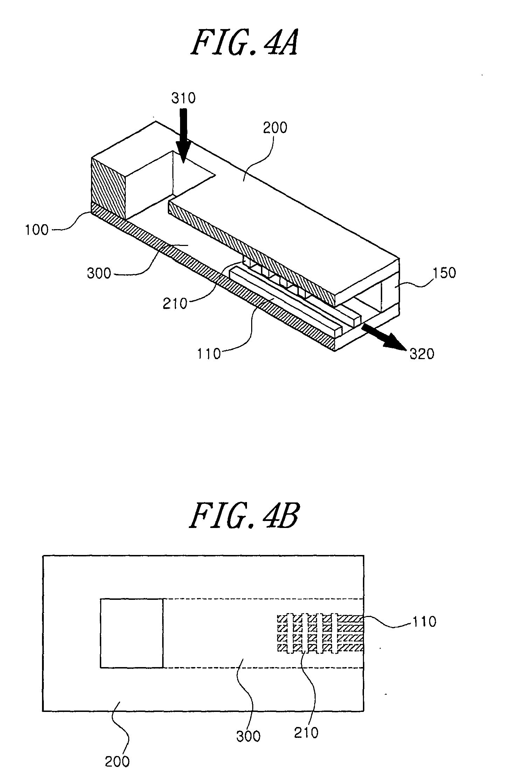

Figs. 4A and 4B are cross sectional view and plan view showing a biosensor having 3D metallic nanowires electrodes in accordance with third embodiment of the present invention.

Referring to Fig. 4A, the biosensor having 3D metallic nanowires electrodes in accordance with the third embodiment of the present invention includes, as like the above embodiments of the present invention, an upper substrate 200 having a plurality of metallic nanowires 210 on a lower surface thereof and an injection opening 310, into which a sample containing biomolecules is injected; a lower substrate 100 having a plurality of metallic nanowires 110 on an upper surface thereof; and a support member 150 supporting the upper and the lower substrates 200 and 100, respectively, with a gap to form a nano channel 300.

As shown in Fig. 4B, one of the metallic nanowires 210 and 110 formed onto the upper and lower substrates 200 and 100, in case of the third embodiment of the present invention, e.g., the metallic nanowires 210 formed onto the upper substrates 200 are vertically disposed to the nano channel 300 and the other, e.g., the metallic nanowires 110 formed onto the lower substrate 100 are parallelly disposed to the nano channel 300. That is, the metallic nanowires 110 of the lower substrate 100 are perpendicularly aligned with the metallic nanowires 210 of the upper substrate 200 when seen from the top.

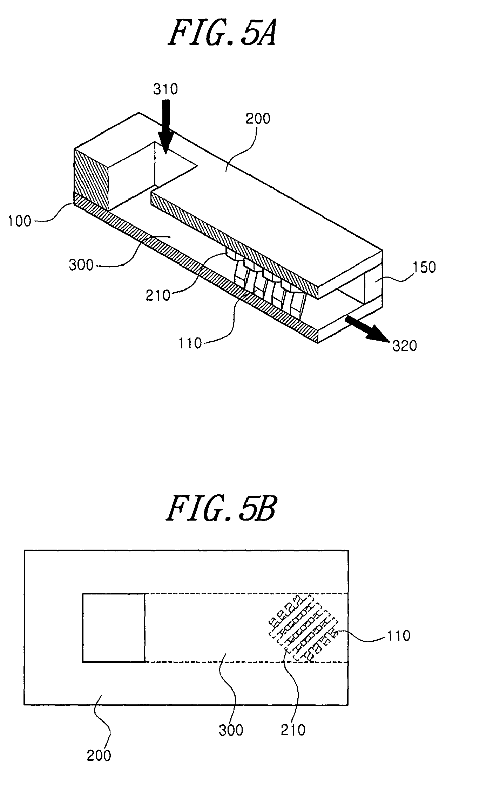

Figs. 5A and 5B are cross sectional view and plan view showing a biosensor having 3D metallic nanowires electrodes in accordance with fourth embodiment of the present invention.

Referring to Fig. 5A, the biosensor having 3D metallic nanowires electrodes in accordance with the fourth embodiment of the present invention includes, as like the above embodiments of the present invention, an upper substrate 200 having a plurality of metallic nanowires 210 on a lower surface thereof and an injection opening 310, into which a sample containing biomolecules is injected; a lower substrate 100 having a plurality of metallic nanowires 110 on an upper surface thereof; and a support member 150 supporting the upper and the lower substrates 200 and 100, respectively, with a gap to form a nano channel 300.

As shown in Fig. 5B, the metallic nanowires 210 and 110 formed onto the upper and lower substrates 200 and 100 are disposed with a predetermined angle to the nano channel 300. That is, the metallic nanowires 110 of the lower substrate 100 are aligned with the metallic nanowires 210 of the upper substrate 200 to have the predetermined angle when seen from the top.

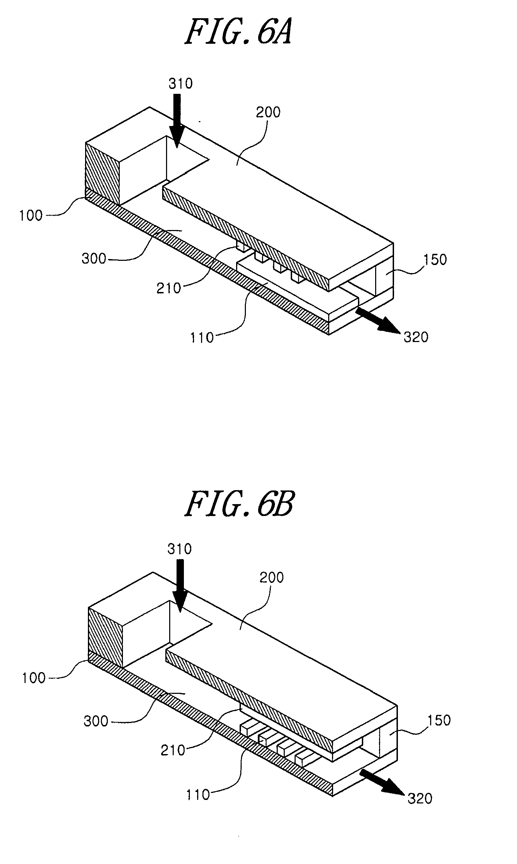

Figs. 6A and 6B are cross sectional view and plan view showing a biosensor having 3D metallic nanowires electrodes in accordance with fifth embodiment of the present invention.

Referring to Fig. 6A, the biosensor having 3D metallic nanowires electrodes in accordance with the fifth embodiment of the present invention includes an upper substrate 200 having a plurality of metallic nanowires 210 on a lower surface thereof and an injection opening 310, into which a sample containing biomolecules is injected; a lower substrate 100 having a metallic electrode 110 on an upper surface thereof; and a support member 150 supporting the upper and the lower substrates 200 and 100, respectively, with a gap to form a nano channel 300.

Referring to Fig. 6B, the biosensor having 3D metallic nanowires electrodes in accordance with the fifth embodiment of the present invention includes an upper substrate 200 having a metallic electrode 210 on a lower surface thereof and an injection opening 310, into which a sample containing biomolecules is injected; a lower substrate 100 having a plurality of metallic nanowires 110 on an upper surface thereof; and a support member 150 supporting the upper and the lower substrates 200 and 100, respectively, with a gap to form a nano channel 300.

That is, in accordance with the fifth embodiment of the present invention, one of electrodes of the upper and the lower substrates 200 and 100 is metallic nanowires, the other is a metallic electrode in a planar shape.

In accordance with the above mentioned embodiments of the present invention, the biosensor having 3D metallic nanowires electrodes includes the upper and the lower substrates units 200 and 100 having a plurality of metallic nanowires and a support member 150 supporting the upper and the lower substrates 200 and 100, respectively, with a gap.

The sample containing the biomolecules to be detected is injected into the injection opening 310 and flows along the 3D metallic nanowires electrodes through the nano channel 300, then exit from a discharge opening 320. In this case, the sample injected from the injection opening 310 can pass through the nano channel without extra pumping due to capillary tube phenomenon of the nano channel.

Meanwhile, the metallic nano wires formed onto the upper and the lower substrates 200 and 100 may be disposed in various configurations, such as vertical, parallel, and predetermined angle to the nano channel so that blocking occurring in the front portion of the nano channel depending on sample to be detected can be reduced or prevented.

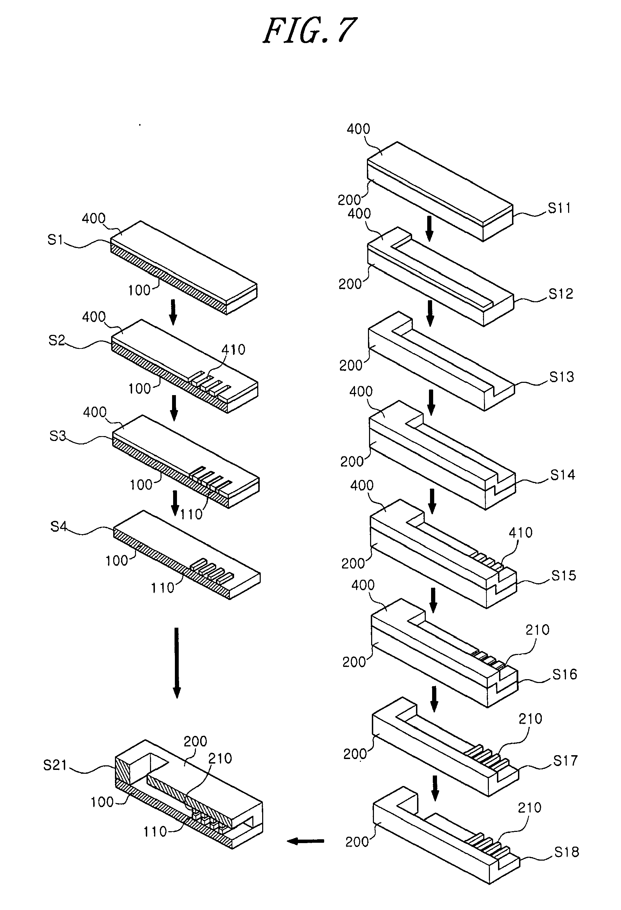

Fig. 7 is a flow diagram showing a manufacturing method of a biosensor having 3D metallic nano wires electrodes in accordance with an embodiment of the present invention.

A resist 400 is coated onto an upper surface of the lower substrate 100 (S1). A pattern 410 is formed in the resist 400 by a nano patterning method such as e-beam lithography and nano imprinting (S2). Location and alignment of the pattern 410 enable to form various alignments of the metallic nanowires 210 and 110 onto the upper and the lower substrate 200 and 100 and to form various shape of the nano channel 300. In the pattern 410 of the lower substrate 100, a metal is deposited to form the metallic nanowires 110 onto the lower substrate 100 (S3). The metallic nanowires 110 of the lower substrate 100 are made of low resistance metal such as Ag, Cu, Au, Al, and Pt or an alloy containing one of those metals. The resist 400 is removed to form the metallic nanowires 110 onto the upper surface of the lower substrate 100 (S4) . Meanwhile, in case of the upper substrate 200, a resist 400 is coated onto a lower surface of the upper substrate 200 (S11). A nano channel is patterned in the resist 400, determining width and length of the nano channel 300 (S12). The upper substrate 200 is etched through the patterned resist 400 as a mask (S13). Depth of the nano channel 300 can be adjusted in the process of the etching. The etching may be performed by any one of the processes among chemical wet etching, VPE(vapor-phase etching), plasma etching, and RIE(Reactive Ion Etching). Thereafter, as like the lower substrate 100, a resist 400 is coated again onto the lower surface of the upper substrate 200 (S14). A pattern 410 is formed in the resist 400 (S15) In the pattern 410 of the upper substrate 200, a metal is deposited (S16). The resist 400 is removed to form the metallic nanowires 210 onto the lower surface of the upper substrate 200 (S17). Then, the injection opening 310 is formed in the upper substrate 200 to inject the sample (S18).

Finally, by using the metallic nanowires 210 and 110 of the upper and the lower substrates 200 and 100, the upper and the lower substrates 200 and 100 are aligned with a optical or mechanical method, then, the aligned substrates 200 and 100 are bonded together (S21). The bonding may be performed by any one of process among anodic bonding, fusion bonding, polymer bonding, and SAM(self assembled monolayer).

A gap distance of the 3D metallic nanowires in accordance with the present invention manufactured through above mentioned processes can be varied by adjusting a depth of the nano channel 300 and a deposition thickness of the upper and the lower metallic nanowires 210 and 110.

Though, the metallic nano wires are deposited on the upper and the lower substrates 200 and 100 in the above embodiments, a planar metallic electrode may be deposited on one of the upper and the lower substrates 200 and 100 and the metallic nano wires may be deposited on the other substrates.

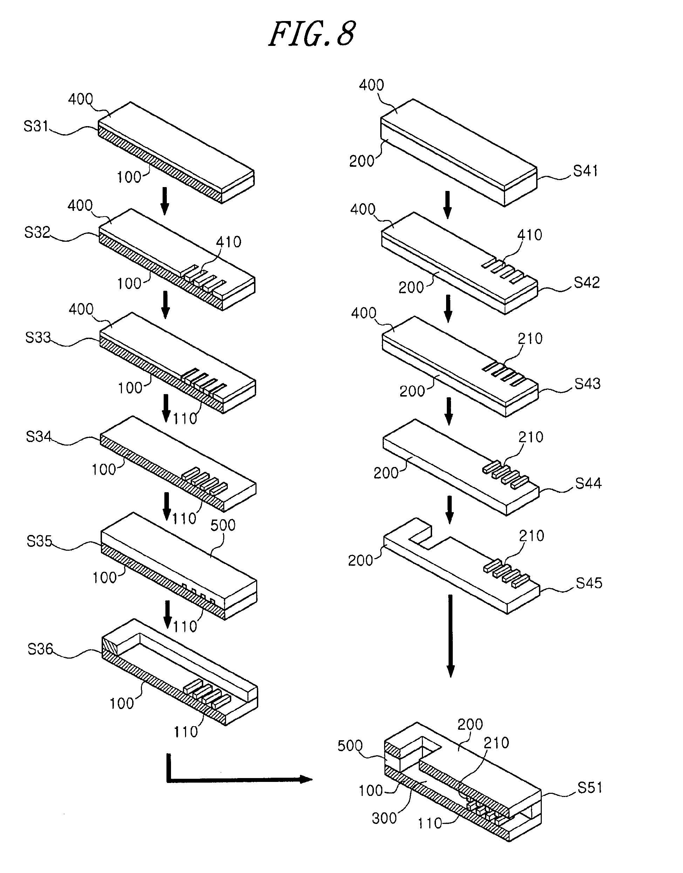

Fig. 8 is a flow diagram showing a manufacturing method of a biosensor having 3D metallic nano wires electrodes in accordance with another embodiment of the present invention.

By using the above mentioned method shown in Fig. 7, metallic nanowires 110 are formed onto the upper surface of the lower substrate 100 (S31 to S34). Metallic nanowires 210 are also formed onto the lower surface of the upper substrate 200 by using the method (S41 to S44). Then, the injection opening 310 is formed in the upper substrate 200 to inject the sample (S45).

A polymer 500 is spin coated onto the upper surface of the lower substrate 100 where the metallic nanowires 110 is formed to for a nano channel 300 (S35). The depth of the nano channel 300 is determined by viscosity of the polymer 500 and RPM(revolutions per minute) and elapsed time of the spin coating.

The polymer 500 is etched through a mask pattern after determining the width and length of the nano channel 300 (S36). The etching may be performed by any one of the processes among chemical wet etching, VPE(vapor-phase etching), plasma etching, and RIE(Reactive Ion Etching).

Finally, by using the metallic nanowires 210 and 110 of the upper and the lower substrates 200 and 100, the upper and the lower substrates 200 and 100 are aligned with a optical or mechanical method, then, the aligned substrates 200 and 100 are bonded together (S51). The bonding may be performed by any one of process among anodic bonding, fusion bonding, polymer bonding, and SAM(self assembled monolayer).

A gap distance of the 3D metallic nanowires in accordance with the present invention manufactured through above mentioned processes can be varied by adjusting a depth of the nano channel 300 and a deposition thickness of the upper and the lower metallic nanowires 210 and 110.

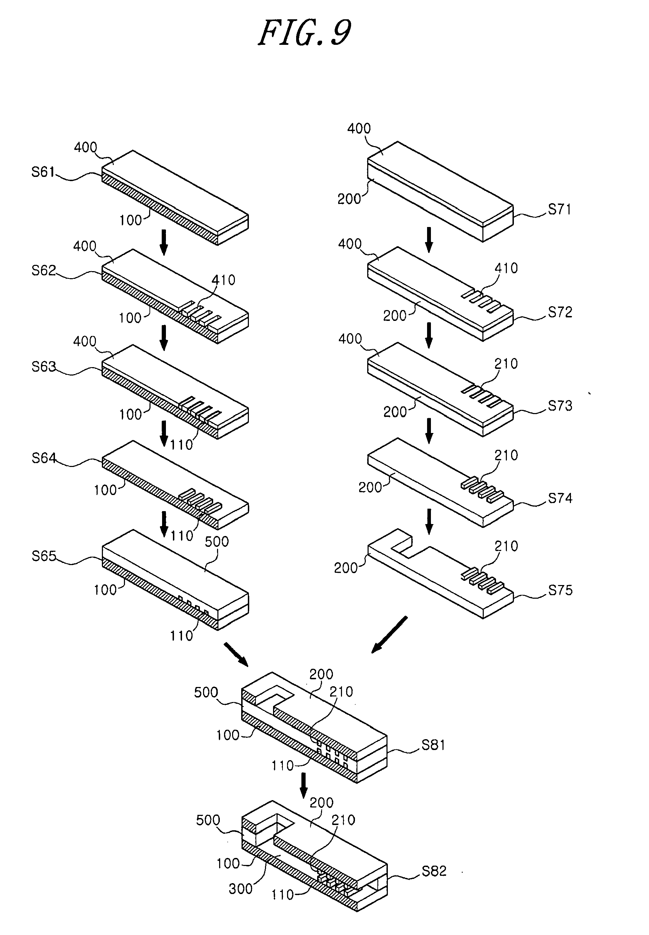

Fig. 9 is a flow diagram showing a manufacturing method of a biosensor having 3D metallic nano wires electrodes in accordance with still another embodiment of the present invention.

By using the above mentioned method referring to Fig. 7, metallic nanowires 110 are formed onto the upper surface of the lower substrate 100 (S61 to S64). Metallic nanowires 210 are also formed onto the lower surface of the upper substrate 200 by using the method (S71 to S74). Then, the injection opening 310 is formed in the upper substrate 200 to inject the sample (S75).

A polymer 500 is spin coated onto the upper surface of the lower substrate 100 where the metallic nanowires 110 is formed to for a nano channel 300 (S65). The depth of the nano channel 300 is determined by viscosity of the polymer 500 and RPM(revolutions per minute) and elapsed time of the spin coating.

By using the metallic nanowires 210 and 110 of the upper and the lower substrates 200 and 100, the upper and the lower substrates 200 and 100 are aligned with an optical or mechanical method, then, the aligned substrates 200 and 100 are bonded together (S81). The bonding may be performed by any one of process among anodic bonding, fusion bonding, polymer bonding, and SAM(self assembled monolayer).

Finally, the polymer 500 of the bonded upper and the lower substrates 200 and 100, the polymer 500 being a support as described above, is exposed by using a mask pattern during the photo lithography after determining the width and length of the nano channel 300, then, the exposed polymer 500 through the injection opening 310 and the discharge opening 320 is removed (S82).

A gap distance of the 3D metallic nanowires in accordance with the present invention manufactured through above mentioned processes can be varied by adjusting a depth of the nano channel 300 and a deposition thickness of the upper and the lower metallic nanowires 210 and 110.

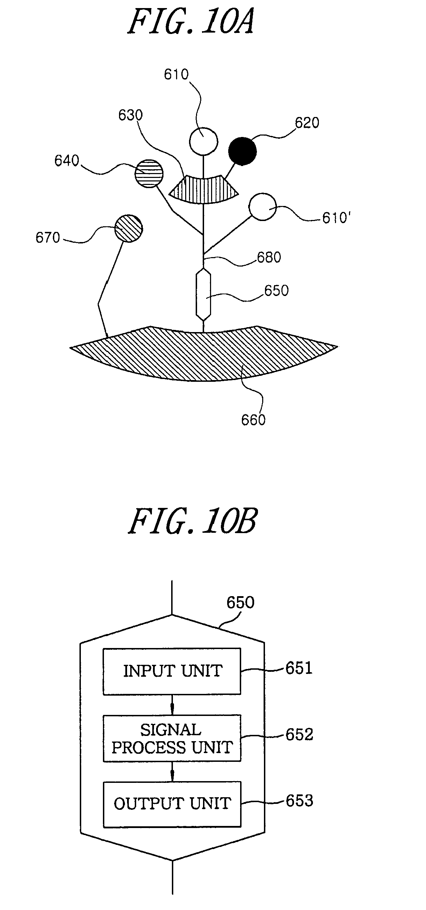

Fig. 10A is a schematic diagram of a bio disk system having the biosensor manufactured in accordance with the present invention.

Referring to Fig. 10A, the bio disk system in accordance with the present invention includes a biosensor having the 3D metallic nanowires electrodes, buffer injection chambers 610 and 610', a sample injection chamber 620, a preprocessing chamber 630, a calibrant injection chamber 640, a discharge chamber 660, a discharge opening 670, and a micro channel 680. Above elements are arranged on a disk type body to form a Lab-on-a-Chip(LOC).

Hereinafter, operational principle of the above bio disk system will be described in detail.

If the disk is rotated after injecting a sample containing the biomolecules to be detected into the sample injection chamber 620, the biosensor 650 is cleaned by the buffer exiting from the lower buffer injection chamber 610' due to the centrifugal force. Then, a signal reference point of the biosensor 650 is calibrated by the calibrant fluid exiting from the calibrant injection chamber 640.

Further, the preprocessing chamber 630 is cleaned by the buffer exiting from the upper buffer injection chamber 610. Then, the sample injected into the sample injection chamber 620 flows into the preprocessing chamber 630 for the preprocessing. The preprocessing is conducted according to types of the biomolecules to be detected using the bio disk system.

The preprocessed sample at the preprocessing chamber 630 flows into the biosensor 650 through the micro channel 680 so that detection procedure using biosensor having the 3D metallic nanowires electrodes in accordance with the present invention is performed.

After the detection procedure, the sample is collected in the discharge chamber 660, then discharged through the discharge opening 670.

Fig. 10B is a schematic diagram of a biosensor having the 3D metallic nanowires electrodes manufactured in accordance with the present invention.

Referring to Fig. 10B, the biosensor 650 included in the above bio disk system includes an input unit 651, a signal process unit 652, and output unit 653. The input unit 651 includes the biosensor in accordance with the present invention. The signal process unit 652 processes the signal from an electrode of the input unit 651. The output unit 653 outputs the signal processed from the signal process unit 652 into a form such as electric, magnetic, and optical signal.

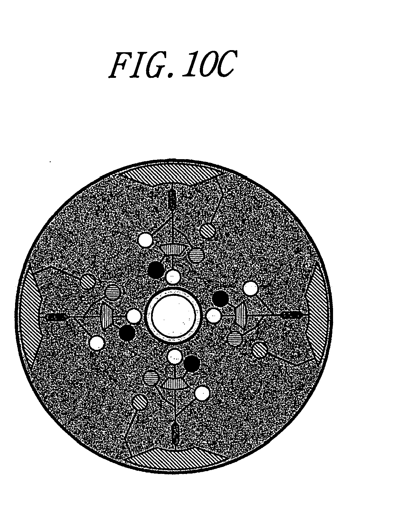

Fig. 10C is a plan view showing the bio disk system shown in Fig. 10A arranged on a disk type body.

Referring to Fig. 10C, one or more of the bio disk system may be disposed on the disk type body as shown in Fig. 10C. The disk type body includes a thin disk body such as CD-ROM, DVD, bio CD, and bio DVD.

While the invention has been shown and described with respect to the embodiments, it will be understood by those skilled in the art that various changes and modifications may be made without departing from the scope of the invention as defined in the following claims.

## Claims

1. A biosensor comprising:
an upper substrate having a plurality of metallic nanowires on a lower surface thereof and an injection opening, into which a sample containing biomolecules is injected;
a lower substrate having a plurality of metallic nanowires on an upper surface thereof; and
a support member supporting the upper and the lower substrates, respectively, with a gap to form a nano channel,
wherein the metallic nanowires on the upper substrate and the lower substrate form 3D metallic nanowires electrodes.

2. The biosensor of claim 1, wherein the metallic nanowires on the upper substrate and the lower substrate are vertically disposed to the nano channel.

3. The biosensor of claim 2, wherein the metallic nanowires on the upper substrate and the lower substrate are aligned with each other.

4. The biosensor of claim 2, wherein the metallic nanowires on the upper substrate and the lower substrate are alternately aligned with each other.

5. The biosensor of claim 1, wherein one of the metallic nanowires on the upper substrate and the lower substrate is vertically disposed to the nano channel, and the other is parallelly disposed to the nano channel..

6. The biosensor of claim 1, wherein the metallic nanowires on the upper substrate and the lower substrate are disposed to have predetermined angles to the nano channel.

7. The biosensor of any one of claims 1 to 6, wherein the metallic nanowires on the upper substrate and the lower substrate are metal selected from the group consisting of Ag, Cu, Au, Al, and Pt or an alloy containing any one of the metal selected from the group.

8. A biosensor comprising:
an upper substrate having a metallic electrode on a lower surface thereof and an injection opening, into which a sample containing biomolecules is injected;
a lower substrate having a metallic electrode on an upper surface thereof; and
a support member supporting the upper and the lower substrates, respectively, with a gap to form a nano channel,
wherein one of electrodes on the upper substrate and the lower substrate is formed with metallic nanowires, and
wherein the metallic nanowires on the upper substrate and the lower substrate form 3D metallic nanowires electrodes.

9. The biosensor of claim 8, wherein the metallic electrodes on the upper substrate and the lower substrate are metal selected from the group consisting of Ag, Cu, Au, Al, and Pt or an alloy containing any one of the metal selected from the group.

10. A method for manufacturing a biosensor comprising:
forming a metallic electrode on an upper surface of a lower substrate;
forming a nano channel pattern in a resist coated onto a lower surface of an upper substrate, determining a width and a length of the nano channel;
etching a nano channel through the nano channel pattern as a mask;
forming a metallic electrode on the etched nano channel;
aligning the upper substrate with the lower substrate by using the metallic electrodes on the upper substrate and the lower substrate; and
bonding the aligned upper substrate and lower substrate.

11. The method for manufacturing a biosensor of claim 1, wherein at least one of the electrodes on the upper substrate and the lower substrate is formed with metallic nanowires.

12. A method for manufacturing a biosensor comprising:
forming metallic nanowires on an upper surface of a lower substrate;
forming metallic nanowires on a lower surface of an upper substrate;
spin-coating a polymer on the upper surface of the lower substrate to form a nano channel;
etching the polymer to form a nano channel through a mask after determining a width and a length of the nano channel;
aligning the upper substrate with the lower substrate by using the metallic nanowires on the upper substrate and the lower substrate; and
bonding the aligned upper substrate and lower substrate.

13. A method for manufacturing a biosensor comprising:
forming metallic nanowires on an upper surface of a lower substrate;
forming metallic nanowires on a lower surface of an upper substrate;
spin-coating a polymer on the upper surface of the lower substrate to form a nano channel;
aligning the upper substrate with the lower substrate by using the metallic nanowires on the upper substrate and the lower substrate;
bonding the aligned upper substrate and lower substrate; and
etching the polymer to form a nano channel through a mask after determining a width and a length of the nano channel.

14. The method for manufacturing a biosensor of any one of claims 10 to 13, wherein the etching is performed by any one of process of chemical wet etching, VPE(vapor-phase etching), plasma etching, and RIE(Reactive Ion Etching).

15. The method for manufacturing a biosensor of any one of claims 10 to 13, wherein the bonding of the upper substrate and the lower substrate is performed by any one of process of anodic bonding, fusion bonding, polymer bonding, and SAM(self assembled monolayer) .

16. The method for manufacturing a biosensor of any one of claims 10 to 1-3, wherein the gap distance of the metallic nanowires or the gap distance of the metallic electrodes of the upper substrate and the lower substrate are varied by adjusting a depth of the nano channel and a deposition thickness of the metallic nanowires or the metallic electrodes.

17. A bio disk system comprising the biosensor of any one of claims 1 to 9 to detect biomolecules from an injected sample.

18. The bio disk system of claim 17, wherein the biosensor is disposed on a thin disk type body of CD-ROM, bio CD, or bio DVD.
